# EUROPEAN PATENT APPLICATION

(11) **EP 1 167 344 A1**
(43) Date of publication of application: **02.01.2002**
(21) Application number: 00909655.3
(22) Date of filing: 15.03.2000
(51) Int. Cl.: C07C 229/34, C07C 227/16, C07C 227/18, C07D 215/56

(54) **PROCESS FOR PRODUCING 5-AMINO-8- ALKYLQUINOLO NECARBOXYLIC ACID DERIVATIVES AND INTERMEDIATES IN THE PRODUCTION THEREOF**

(30) Priority: 17.03.1999 JP 7190999; 23.06.1999 JP 17621999
(71) Applicant: DAIICHI PHARMACEUTICAL CO., LTD., Chuo-ku, Tokyo 103-0027 (JP)
(72) Inventor: TAKEMURA, Makoto, Daiichi Pharmaceutical Co.,Ltd., Edogawa-ku, Tokyo 134-0081 (JP); TAKAHASHI, Hisashi, Daiichi Pharmaceutical Co.,Ltd, Edogawa-ku, Tokyo 134-0081 (JP)
(74) Representative: Kinzebach, Werner, Dr.
(86) International application number: JP0001582
(87) International publication number: WO0055116

(57) **Abstract**

The present invention provides a process for producing intermediates of antibacterial agents in accordance with the following steps and intermediates in the production thereof. 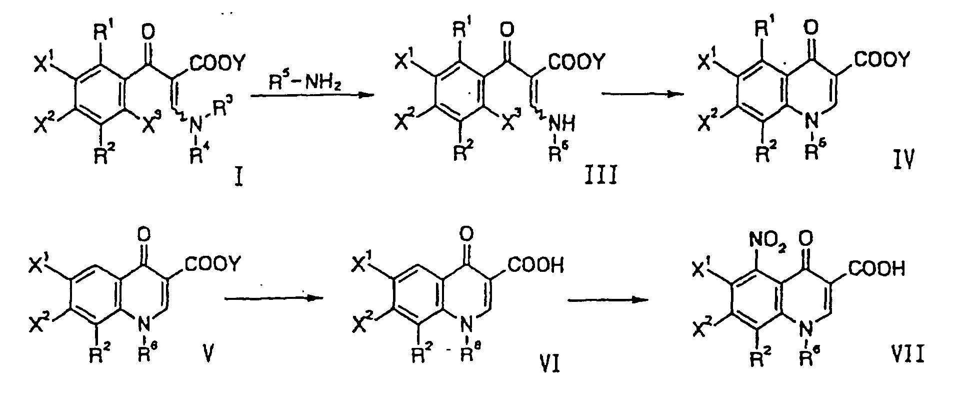 (wherein R¹: hydrogen atom or nitro group; R²: an alkyl group having 1 to 6 carbon atoms; R³, R⁴: an alkyl group having 1 to 6 carbon atoms or may form a 5-membered or 6-membered cyclic structure in combination with the nitrogen atom to which they are bonded, and the cyclic structure may contain oxygen atom, nitrogen atom, sulfinyl group, or sulfonyl group as a ring-constituting element; R⁶: a halogenocycloalkyl group having 3 to 6 carbon atoms; X¹ represents a halogen atom or hydrogen atom; X², X³: a leaving group; and Y: an alkyl group having 1 to 6 carbon atoms or an alkenyl group having 2 to 6 carbon atoms)

## Description

### TECHNICAL FIELD

The present invention relates to a novel and efficient process for producing a 5-amino-1,4-dihydro-8-alkyl-4-oxoquinoline-3-carboxylic acid derivative having a substituent at the 7-position and an intermediate in the production thereof.

### BACKGROUND ART

Since the discovery of norfloxacin, fluoroquinolone-type synthetic antibacterial agents have developed as chemotherapeutic agents effective for almost systemic infectious diseases through the improvement of the antibacterial activity and pharmacokinetics, and a number of compounds have been provided for clinical use.

Among them, 5-amino-1,4-dihydro-8-alkyl-4-oxoquinoline-3-carboxylic acid derivatives having a substituent at the 7-position exhibit excellent antibacterial activity, pharmacokinetics, safety, and the like, and are useful as quinolone-type synthetic antibacterial agents (cf. JP-A-7-309864 (the term "JP-A" as used herein means "unexamined published Japanese patent application"), WO97/19072, and so forth). For the production of these compounds, 5-amino-1,4-dihydro-8-alkyl-4-oxoquinoline-3-carboxylic acids having a leaving group at the 7-position are used as intermediates for the production (cf. above-mentioned publications).

Such 5-amino-1,4-dihydro-8-alkyl-4-oxoquinoline-3-carboxylic acids, for example, 5-amino-1-eyelopropyl-6,7-difluoro-1,4-dihydro-8-methyl-4-oxoquinoline-3-carboxylic acid is produced from 2,4,5-trifluoro-3-methylbenzoic acid according to the method shown below (Conventional method 1), the detail of which is described in JP-A-7-309864, JP-A-8-198819, JP-A-8-259561, and Chemical & Pharmaceutical Bulletin; Vol. 44, p. 1074 (1996).

### Conventional method 1

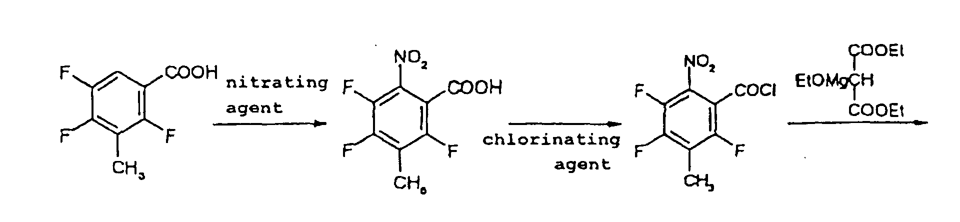

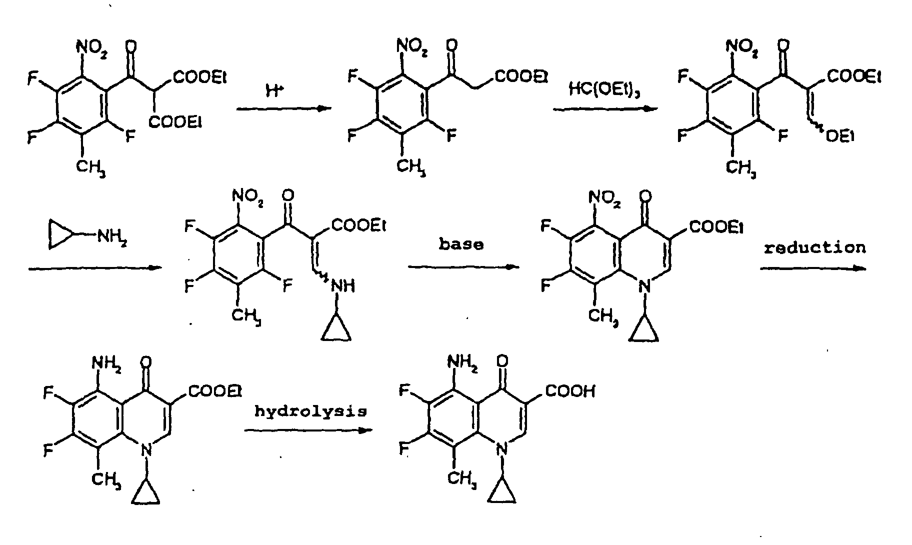

Moreover, 5-amino-6,7-difluoro-1-[2-(S)-fluoro-1-(R)-cyclopropyl]-1,4-dihydro-8-methyl-4-oxoquinoline-3-carboxylic acid is produced from 2,4,5-trifluoro-3-methylbenzoic acid according to the method shown below (Conventional method 2), the detail of which is described in JP-A-8-277284.

### Conventional method 2

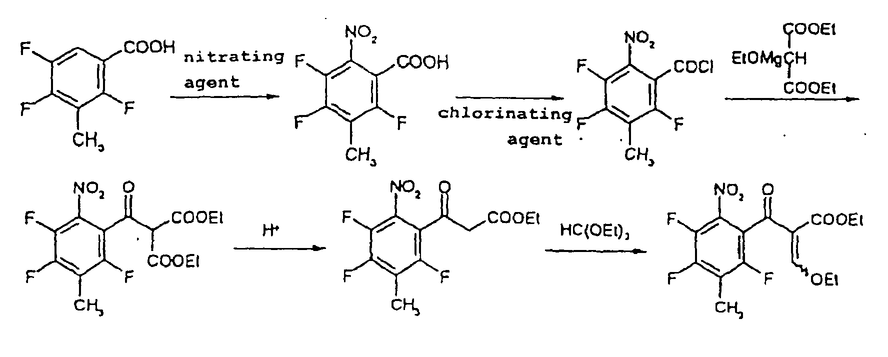

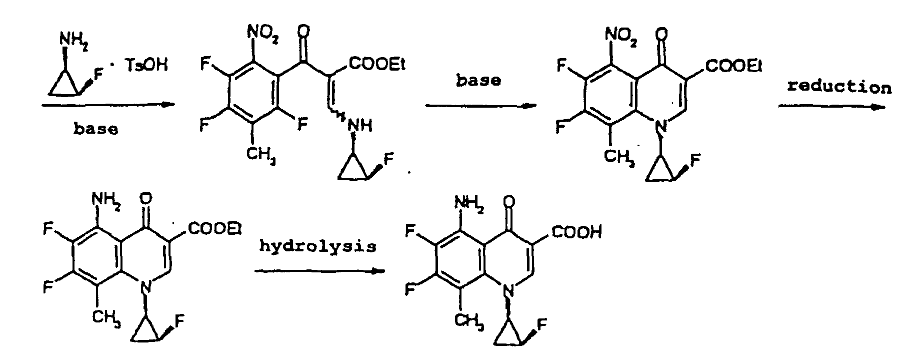

Furthermore, 5-amino-1-cyclopropyl-6,7-difluoro-1,4-dihydro-8-methyl-4-oxoquinoline-3-carboxylic acid is produced according to the method of nitration of 1-cyclopropyl-6,7-difluoro-1,4-dihydro-8-methyl-4-oxoquinoline-3-carboxylic acid at the 5-position and successive reduction shown below (Conventional method 3), the detail of which is described in JP-A-62-215572. Conventional method 3

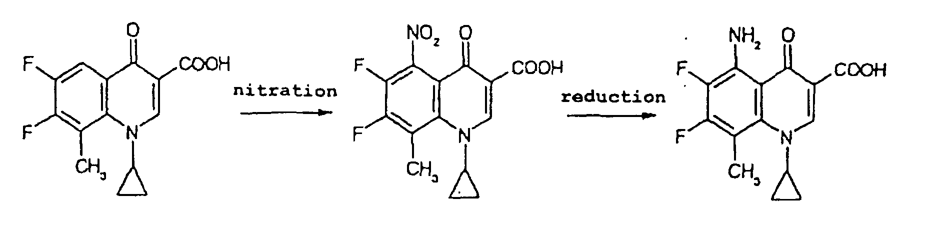

However, Conventional methods 1 and 2 include many steps and thus the curtailment of the synthetic steps has been desired for industrial production. Furthermore, in Conventional method 3, it has been known that nitration of 1-cyclopropyl-6,7-difluoro-1,4-dihydro-8-methyl-4-oxoquinoline-3-carboxylic acid at the 5-position proceeds in very low yields.

On the other hand, JP-A-63-316757 describes a compound represented by the following formula as an intermediate useful for fluoroquinolonecarboxylic acid synthetic antibacterial agents having a lower alkoxyl group at the 8-position 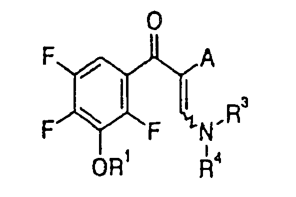 [wherein R¹ represents a lower alkyl group, R² and R³ represent the same or different alkyl groups, or may represent a cyclic amino group formed in combination with the nitrogen atom to which they are bonded and optionally in combination with oxygen atom, sulfur atom, sulfinyl group or sulfonyl group, and A represents nitrile group or a lower alkoxycarbonyl group (by the way, the definitions of the substituents in this formula are not relevant to the definitions of the invented compounds of the present application)].

Furthermore, JP-A-5-320107 describes a compound represented by the following formula as an intermediate useful for fluoroquinolonecarboxylic acid synthetic antibacterial agents having oxygen atom at the position corresponding to the 8-position of the fluoroquinolonecarboxylic acid skeleton 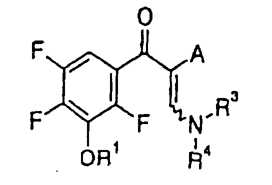 [wherein R¹ represents benzyl group, R² and R³ represent the same or different alkyl groups, and A represents -COOR⁵ (wherein R⁵ represents a lower alkyl group or an allyl group) (by the way, the definitions of the substituents in this formula are not relevant to the definitions of the invented compounds of the present application)].

However, these publications do not describe the compound of the invention represented by the formula (I) [hereinafter, the compound is abbreviated as Compound (I), and also, the compounds having different number are similarly abbreviated as above], and the process for producing 1,4-dihydro-8-alkyl-5-nitro-4-oxoquinoline-3-carboxylic acid ester derivatives using it as an intermediate for the production has also not been known.

Furthermore, 3-dialkylamino-2-benzoylacrylic acid ester derivatives produced by reacting benzoyl halides with 3-dialkylamnoacrylic acid ester derivatives are known to be useful intermediates for producing 1,4-dihydro-4-oxoquinoline-3-carboxylic acid ester derivatives in short steps (cf. EP Publication No. 300311).

And, 3-dialkylamino-2-(3-methylbenzoyl)acrylic acid ester derivatives which may be used as intermediates for the production of 1,4-dihydro-8-methyl-4-oxoquinoline-3-carboxylic acids are described in JP-A-7-101918.

This publication describes a compound represented by the following formula: 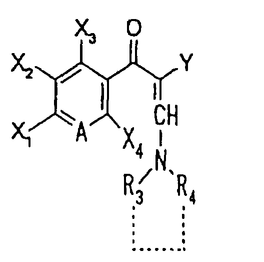 [wherein Y represents a nitrile, an ester group -COOR₁ (wherein R₁ represents C₁-C₄ alkyl group), or acetyl group, A represents nitrogen or C-R₂ (wherein R₂ represents hydrogen, methyl, a halogen, nitro, methoxy, or cyano), X₁ and X₂ may be the same or different and represent halogen atoms, X₃ represents hydrogen, a halogen or nitro group, X₄ represents a halogen, nitro, methoxy or methylthio, and R₃ and R₄ may be the same or different and represent alkyl groups having 1 to 4 carbon atoms or form 5-membered or 6-membered ring together with the nitrogen atom to which they are bonded and the ring may additionally contain an atom -O- or -S- or a group -SO₂-. (The definitions of these substituents are not relevant to the definitions of the symbols used in the present invention even when the same symbols are used)]. The publication also describes a process for producing a compound represented by the following formula: 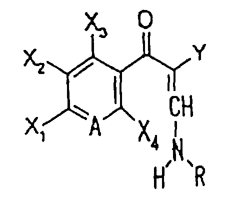 [wherein Y, A, X₁, X₂, X₃ and X₄ are the same as described in the above formula, and R represents an alkyl having 1 to 6 carbon atoms, 2-fluoroethyl, 2-chloroethyl, 2-hydroxyethyl, 1-(hydroxymethyl)ethyl, cyclopropyl, methoxy, 4-fluorophenyl, dimethylamino, formylmethylamino or isopropylideneamino. (The definitions of these substituents are not relevant to the definitions of the symbols used in the present invention even when the same symbols are used)].

However, there also exists no disclosure of Compound (I) of the invention therein and no description of the process of the invention for producing a 1,4-dihydro-8-alkyl-4-oxoquinoline-3-carboxylic acid ester derivative having a leaving group at the 7-position. Furthermore, the process described therein is different from the process of the invention.

As mentioned above, there is no publication wherein the 3-dialkylamino-2-(3-methylbenzoyl)acrylic acid derivative of the invention is specifically disclosed and also there is no publication wherein the process for producing a 1,4-dihydro-8-alkyl-4-oxoquinoline-3-carboxylic acid ester derivative having a leaving group at the 7-position using the above compound as an intermediate for the production is specifically disclosed.

Furthermore, a 1-halogenocycloalkyl-1,4-dihydro-8-alkyl-5-nitro-4-oxoquinoline-3-carboxylic acid derivative produced by nitration of 1-halogenocycloalkyl-1,4-dihydro-8-alkyl-4-oxoquinoline-3-carboxylic acid at the 5-position, and the process for producing a 5-amino-1-halogenocycloalkyl-1,4-dihydro-8-alkyl-4-oxoquinoline-3-carboxylic acid derivative using it as an intermediate for the production are entirely not known.

Object of the invention is to provide a process for producing efficiently a 5-amino-1,4-dihydro-8-alkyl-4-oxoquinoline-3-carboxylic acid derivative having a leaving group at the 7-position, which is an intermediate for the production of a 5-amino-1,4-dihydro-8-alkyl-4-oxoquinoline-3-carboxylic acid derivative having a substituent at the 7-position useful as a fluoroquinolone-type synthetic antibacterial agent exhibiting excellent antibacterial activity, pharmacokinetics and safety. Specifically, the object is to provide a 3-dialkylamino-2-(3-methylbenzoyl)acrylic acid derivative and a 1,4-dihydro-8-alkyl-5-nitro-4-oxoquinoline-3-carboxylic acid derivative which are intermediates for the production of a 5-amino-1,4-dihydro-8-alkyl-4-oxoquinoline-3-carboxylic acid derivative. Furthermore, the object is to provide a convenient and efficient process for producing a 5-amino-1,4-dihydro-8-alkyl-4-oxoquinoline-3-carboxylic acid derivative using the above derivatives as intermediates in the production thereof.

### DISCLOSURE OF THE INVENTION

As a result of the extensive studies, the inventors of the present application have found a convenient process for producing a 1,4-dihydro-8-alkyl-4-oxoquinoline-3-carboxylic acid ester using the 3-dialkylamino-2-(3-methylbenzoyl)acrylic acid ester derivative described below as an intermediate for the production thereof, which requires short steps and no operation for isolating intermediates.

Moreover, they have also found a process for producing a 1-halogenocycloalkyl-1,4-dihydro-8-alkyl-5-nitro-4-oxoquinoline-3-carboxylic acid derivative through nitration of a l-halogenocycloalkyl-1,4-dihydro-8-alkyl-4-oxoquinoline-3-carboxylic acid derivative (cf. Japanese Patent No. 2714597) at the 5-position, the latter derivative being produced from a 1-halogenocycloalkyl-1,4-dihydro-8-alkyl-4-oxoquinoline-3-carboxylic acid ester derivative.

Furthermore, they have found a novel and efficient process for producing a 5-amino-1-halogenocycloalkyl-1,4-dihydro-8-alkyl-4-oxoquinoline-3-carboxylic acid using a 1-halogenocycloalkyl-1,4-dihydro-8-alkyl-5-nitro-4-oxoquinoline-3-carboxylic acid derivative as an intermediate.

The present invention has been accomplished based on these findings. 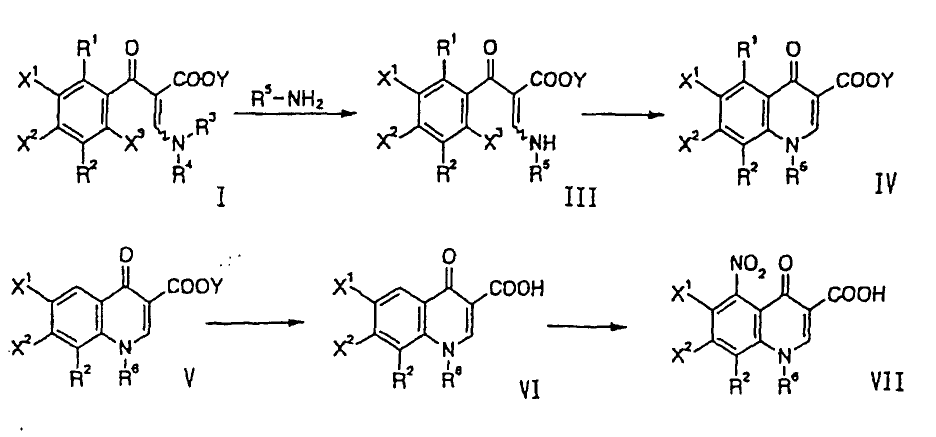 (In the scheme, R¹ represents hydrogen atom or nitro group, R² represents an alkyl group having 1 to 6 carbon atoms,
R³ and R⁴ represent each independently an alkyl group having 1 to 6 carbon atoms, or may form a 5-membered or 6-membered cyclic structure in combination with the nitrogen atom to which they are bonded, and the cyclic structure may contain oxygen atom, nitrogen atom, sulfinyl group, or sulfonyl group as a ring-constituting element,
X¹ represents a halogen atom or hydrogen atom,
X² and X³ represent each independently a leaving group,
Y represents an alkyl group having 1 to 6 carbon atoms or an alkenyl group having 2 to 6 carbon atoms,
R⁵ represents an alkyl group having 1 to 6 carbon atoms, an alkenyl group having 2 to 6 carbon atoms, a halogenoalkyl group having 1 to 6 carbon atoms, a cycloalkyl group having 3 to 6 carbon atoms, a halogenocycloalkyl group having 3 to 6 carbon atoms, an alkoxyl group having 1 to 6 carbon atoms, an alkylamino group having 1 to 6 carbon atoms, an aryl group which may have substituent(s), or a heteroaryl group which may have substituent(s), and
R⁶ represents a halogenocycloalkyl group having 3 to 6 carbon atoms.)

Namely, the present invention relates to a compound represented by the formula (I): 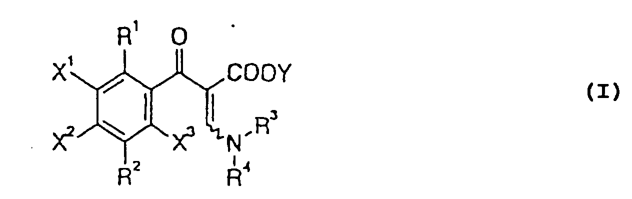 (wherein R¹ represents hydrogen atom or nitro group,
R² represents an alkyl group having 1 to 6 carbon atoms,
R³ and R⁴ represent each independently an alkyl group having 1 to 6 carbon atoms, or may form a 5-membered or 6-membered cyclic structure in combination with the nitrogen atom to which they are bonded, and the cyclic structure may contain oxygen atom, nitrogen atom, sulfinyl group, or sulfonyl group as a ring-constituting element,
X¹ represents a halogen atom or hydrogen atom,
X² and X³ represent each independently a leaving group, and
Y represents an alkyl group having 1 to 6 carbon atoms or an alkenyl group having 2 to 6 carbon atoms) and salt thereof, and also relates to the following. That is, Compound (I) and salt thereof, wherein the leaving group is a halogen atom, a phenylsulfonyl group which may have substituent(s), or an alkylsulfonyl group having 1 to 3 carbon atoms which may have substituent(s);
the above compound and salt thereof, wherein R¹ is nitro group;
the above compound and salt thereof, wherein R¹ is hydrogen atom;
the above compound and salt thereof, wherein R² is methyl group;
the above compound and salt thereof, wherein X¹, X² and X³ are each fluorine atom;
and the like.

Furthermore, the present invention also relates to a process for producing a compound represented by the formula (IV) : 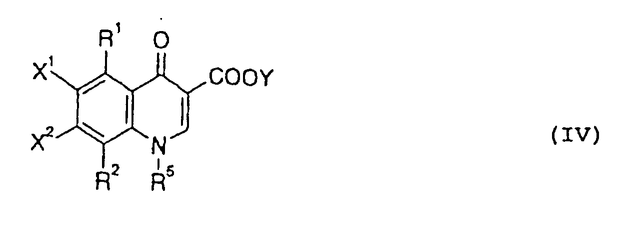 (wherein R¹, R², R⁵, X¹, X² and Y are each the same as defined above),
which comprises reacting a compound represented by the formula (I): 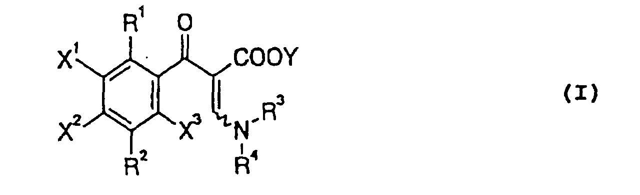 (wherein R¹, R², R³, R⁴, X¹, X², X³ and Y are each the same as defined above) or a salt thereof, with a compound represented by the formula (II) :

R⁵-NH₂ (II)

(wherein R⁵ represents an alkyl group having 1 to 6 carbon atoms, an alkenyl group having 2 to 6 carbon atoms, a halogenoalkyl group having 1 to 6 carbon atoms, a cycloalkyl group having 3 to 6 carbon atoms, a halogenocycloalkyl group having 3 to 6 carbon atoms, an alkoxyl group having 1 to 6 carbon atoms, an alkylamino group having 1 to 6 carbon atoms, an aryl group which may have substituent(s), or a heteroaryl group which may have substituent(s)) to obtain a compound represented by the formula (III): 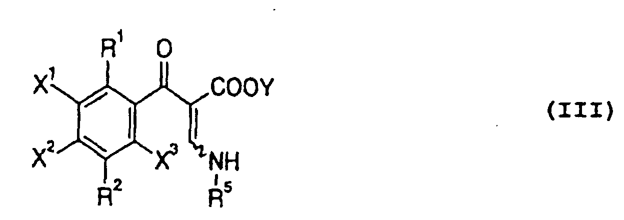 (wherein R¹, R², R⁵, X¹, X², X³ and Y are each the same as defined above) or a salt thereof, and treating the compound of formula (III) with a base. And the present invention also relates to the following. The above process, wherein the leaving group is a halogen atom, a phenylsulfonyl group which may have substituent(s), or an alkylsulfonyl group having 1 to 3 carbon atoms which may have substituent(s);
the above process, wherein R¹ is nitro group;
the above process, wherein R¹ is hydrogen atom;
the above process, wherein R² is methyl group;
the above process, wherein X¹, X² and X³ are each fluorine atom;
the above process, wherein X¹, X² and X³ are each fluorine atom and R⁵ is cyclopropyl group or a halogenocyclopropyl group;
the above process, wherein X¹, X² and X³ are each fluorine atom and R⁵ is 2-(S)-fluoro-1-(R)-cyclopropyl group; and the like.

And also, the invention relates to a compound represented by the formula (VII): 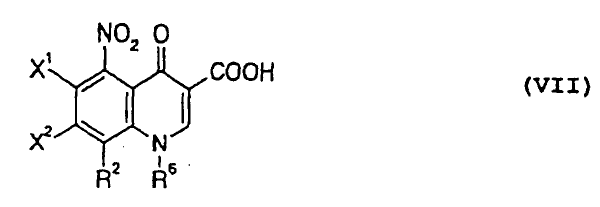 (wherein R⁶ represents a halogenocycloalkyl group having 3 to 6 carbon atoms, and R², X¹ and X² are the same as described above) and salt thereof. Further, the invention also relates to the following. The above compound and salt thereof, wherein the leaving group is a halogen atom, a phenylsulfonyl group which may have substituent(s), or an alkylsulfonyl group having 1 to 3 carbon atoms which may have substituent(s);
the above compound and salt thereof, wherein R² is methyl group;
the above compound and salt thereof, wherein X¹ and X² are each fluorine atom;
the above compound and salt thereof, wherein X¹ and X² are each fluorine atom and R⁶ is 2-(S)-fluoro-1-(R)-cyclopropyl group; and the like.

And the invention relates to a process for producing a compound represented by the formula (VII): 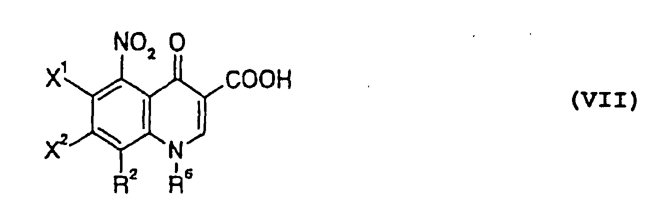 (wherein R², R⁶, X¹ and X² are each the same as defined above) or salt thereof,
which comprises converting the carboxylic acid ester at the 3-position in a compound represented by the formula (V) : 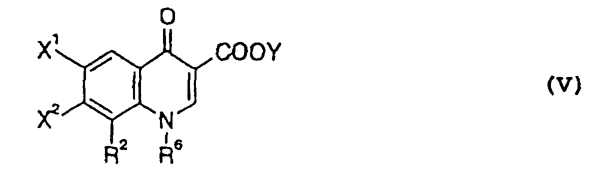 (wherein R², R⁶, X¹, X² and Y are each the same as defined above) or salt thereof, into carboxyl group to obtain a compound represented by the formula (VI) : 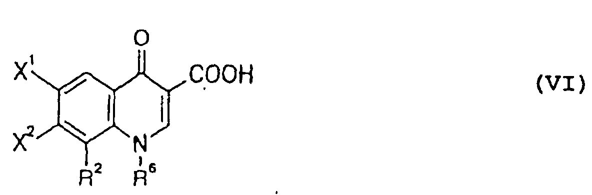 (wherein R², R⁶, X¹ and X² are each the same as defined above) and a salt thereof, and treating the compound of the formula (VI) with a nitrating agent. Further, the invention also relates to the following.

A process for producing a compound represented by the formula (VII): 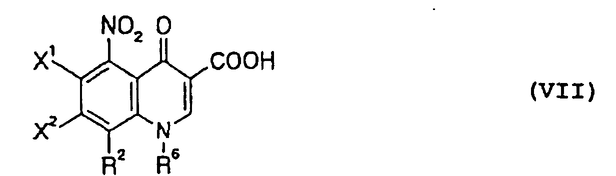 (wherein R², R⁶, X¹ and X² are each the same as defined above) or salt thereof,
which comprises treating a compound represented by the formula (VI): 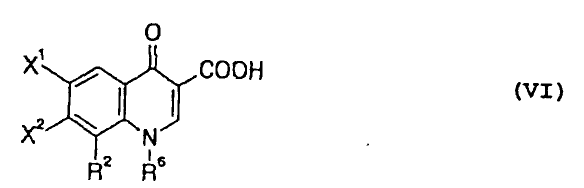 (wherein R², R⁶, X¹ and X² are each the same as defined above) or salt thereof, with a nitrating agent;
the above process, wherein the nitrating agent is nitric acid, a mixture of nitric acid and sulfuric acid, a metal nitrate, acetyl nitrate, dinitrogen pentoxide, or a nitronium salt;
the above process, wherein the nitrating agent is a nitronium salt;
a process for producing a compound represented by the formula (VIII): 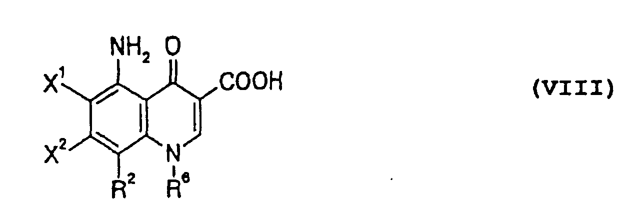
(wherein R², R⁶, X¹ and X² are each the same as defined above) or salt thereof,
which comprises converting the nitro group of a compound represented by the formula (VII): 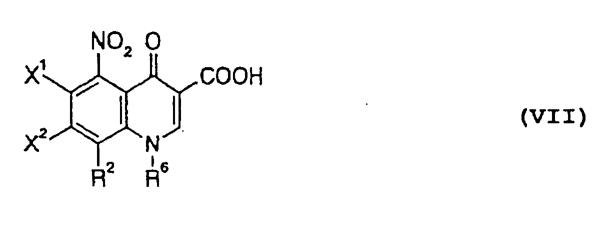 (wherein R², R⁶, X¹ and X² are each the same as defined above) or salt thereof, into amino group;
the above process, wherein the leaving group is a halogen atom, a phenylsulfonyl group which may have substituent(s), or an alkylsulfonyl group having 1 to 3 carbon atoms which may have substituent(s);
the above process, wherein R² is methyl group;
the above process, wherein X¹ and X² are each fluorine atom;
the above process, wherein X¹ and X² are each fluorine atom and R⁶ is 2-(S)-fluoro-1-(R)-cyclopropyl group;
the above process, wherein R³ and R⁴ are each an alkyl group having 1 to 6 carbon atoms;
and the like.

### (Mode for Carrying Out the Invention)

The following will describe each substituent of the compound represented by the formula (I): 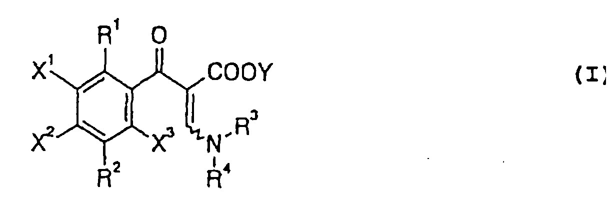 (wherein R¹ represents hydrogen atom or nitro group,
R² represents an alkyl group having 1 to 6 carbon atoms, R³ and R⁴ represent each independently an alkyl group having 1 to 6 carbon atoms, or may form a 5-membered or 6-membered cyclic structure in combination with the nitrogen atom to which they are bonded, and the cyclic structure may contain oxygen atom, nitrogen atom, sulfinyl group, or sulfonyl group as a ring-constituting element,
X¹ represents a halogen atom or hydrogen atom,
X² and X³ represent each independently a leaving group,
Y represents an alkyl group having 1 to 6 carbon atoms or an alkenyl group having 2 to 6 carbon atoms) provided by the present invention.

Substituent R¹ is hydrogen atom or nitro group.

Substituent R² is an alkyl group having 1 to 6 carbon atoms, and the alkyl group includes methyl group, ethyl group, n-propyl group and i-propyl group. Among them, preferred is methyl group.

Substituents R³ and R⁴ represent the same or different alkyl groups having 1 to 6 carbon atoms, or may form a 5-membered or 6-membered cyclic structure in combination with the nitrogen atom to which they are bonded, and the cyclic structure may contain oxygen atom, nitrogen atom, sulfinyl group, or sulfonyl group as a ring-constituting element. The alkyl groups may be linear or branched ones having 1 to 6 carbon atoms.

In the case that R³ and R⁴ form a 5-membered or 6-membered cyclic structure together with the nitrogen atom to which they are bonded, the ring may be either saturated or unsaturated. Among them, preferred is the case of a saturated ring, and the case of a ring structure comprising methylene chain can be exemplified. And this ring may contain the ring-constituting element mentioned above. Examples of the cyclic structure include pyrrolidinyl, piperidino, morpholino, and thiomorpholino.

Substituents R³ and R⁴ are preferably each an alkyl group having 1 to 6 carbon atoms, and examples include methyl group, ethyl group, n-propyl group and i-propyl group. Among these substituents, methyl group and ethyl group are particularly preferred. Further, it is preferred that the substituents R³ and R⁴ are the same.

Substituent X¹ represents a halogen atom or hydrogen atom, but in the case of a halogen atom, fluorine atom is preferred. Substituent X¹ is preferably fluorine atom or hydrogen atom.

Substituents X² and X³ mean each a leaving group. The leaving group includes a halogen atom, a phenylsulfonyl group which may have substituent(s), an alkylsulfonyl group having 1 to 3 carbon atoms which may have substituent(s), and the like. By the way, the leaving group is not limited thereto as far as it is the substituent known as one having a function as a leaving group.

The halogen atom is preferably fluorine atom, chlorine atom, and bromine atom. Examples of the phenylsulfonyl group which may have substituent(s) include benzenesulfonyl group and p-toluenesulfonyl group, and examples of the alkylsulfonyl group having 1 to 3 carbon atoms which may have substituent(s) include methanesulfonyl group, trifluoromethanesulfonyl group, and the like.

Substituents X² and X³ are preferably each fluorine atom, chlorine atom and bromine atom, and particularly preferred is fluorine atom.

Substituent Y represents an alkyl group having 1 to 6 carbon atoms or an alkenyl group having 2 to 6 carbon atoms. Among them, the alkyl group may be linear or branched one having 1 to 6 carbons, and preferred are methyl group, ethyl group, n-propyl group and i-propyl group. Of these substituents, methyl group and ethyl group are particularly preferred. As the alkenyl group, allyl group may be mentioned. As the substituent Y, particularly preferred is ethyl group.

Compound (I) can be produced according to the following steps. 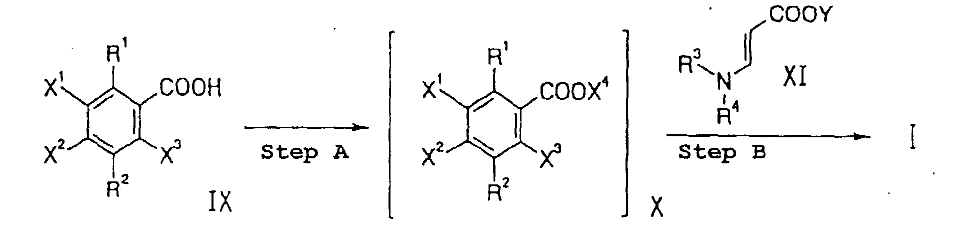 (In the scheme, R¹, R², R³, R⁴, X¹, X², X³ and Y are each the same as defined above.
X⁴ represents a halogen atom but is preferably chlorine atom or bromine atom.)

In Step A, a substituted benzoyl halide compound represented by the formula (X), especially a chloride is synthesized according to an ordinarily known method for preparing an acid halide, using Compound (IX) such as 2,4,5-trifluoro-3-methyl-6-nitrobenzoic acid which is a known compound, as a starting material. The thus obtained Compound (X) may be isolated, but can be treated, without isolation, with Compound (XI) in a solvent in the presence of a base in successive Step B to produce the compound (I).

Compound (XI) may be employed as commercially available one, and may be used in an amount of 1 to 1.2 equivalents to the compound (X) for the reaction. The solvent usable in Step B may be any solvent which is inert under the reaction conditions. Examples thereof include ether solvents such as tetrahydrofuran, diethyl ether, 1,4-dioxane and dimethoxyethane; aromatic hydrocarbon solvents such as benzene, toluene and xylene; halogenated hydrocarbon solvents such as methylene chloride and dichloroethane; or mixtures thereof. The base usable in Step B includes aromatic nitrogen-containing heterocyclic compounds and aromatic or aliphatic tertiary organic base compounds such as pyridine, triethylamine and N-methylpiperidine. Preferred is triethylamine. Reaction temperature for Step B may be usually in the range of room temperature to 200°C, and the reaction can be preferably carried out in the temperature range of 20°C to 150°C. Reaction time may be in the range of 30 minutes to 48 hours, and the reaction is usually completed within the range of about 30 minutes to 12 hours.

Step A and Step B can be carried out as a successive step in the same reaction vessel without isolating the product in Step A. As a method for isolating Compound (I), a general method can be mentioned wherein a water-insoluble organic solvent and water are added to a reaction mixture after its concentration or without its concentration to remove formed salt into the aqueous layer. Then, the organic layer is concentrated and the solvent is removed to obtain a product of a high purity. By the way, for further purification, a general purification method such as column chromatography or recrystallization may be applied, whereby the product can be isolated as a pure substance.

The following scheme shows the process for producing a 1,4-dihydro-8-alkyl-4-oxoquinoline-3-carboxylic acid esters, using Compound (I) as an intermediate for the production. 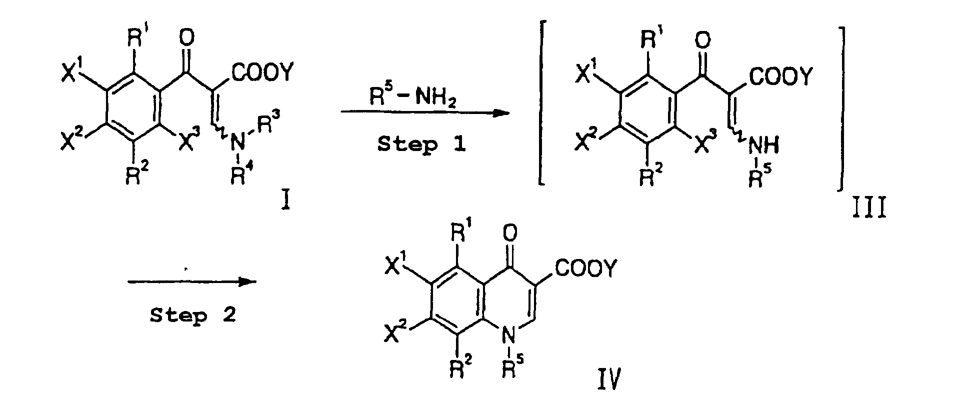 (In the scheme, R¹ represents hydrogen atom or nitro group, R² represents an alkyl group having 1 to 6 carbon atoms, R³ and R⁴ represent each independently an alkyl group having 1 to 6 carbon atoms, or may form a 5-membered or 6-membered cyclic structure in combination with the nitrogen atom to which they are bonded, and the cyclic structure may contain oxygen atom, nitrogen atom, sulfinyl group, or sulfonyl group as a ring-constituting element,
X¹ represents a halogen atom or hydrogen atom,
X² and X³ represent each independently a leaving group,
Y represents an alkyl group having 1 to 6 carbon atoms or an alkenyl group having 2 to 6 carbon atoms,
R⁵ represents an alkyl group having 1 to 6 carbon atoms, an alkenyl group having 2 to 6 carbon atoms, a halogenoalkyl group having 1 to 6 carbon atoms, a cycloalkyl group having 3 to 6 carbon atoms, a halogenocycloalkyl group having 3 to 6 carbon atoms, an alkoxyl group having 1 to 6 carbon atoms, an alkylamino group having 1 to 6 carbon atoms, an aryl group which may have substituent(s), or a heteroaryl group which may have substituent(s), and
R⁶ represents a halogenocycloalkyl group having 3 to 6 carbon atoms.)

Hereinafter, each reaction will be explained in detail according to the above scheme by exemplifying the process for producing ethyl 6,7-difluoro-1-[2-(S)-fluoro-1-(R)-cyclopropyl]-1,4-dihydro-8-methyl-4-oxoquinoline-3-carboxylate which is included in 1,4-dihydro-8-alkyl-4-oxoquinoline-3-carboxylic acid esters, but the process of the present invention is not limited to this example.

In Step 1, Compound (I) of the invention and Compound (II) are reacted in a solvent to effect amine exchange, whereby Compound (III) can be obtained. Compound (III) thus obtained can be isolated according to an ordinary method, but Step 2 may be successively carried out without isolating the compound depending on the solvent used.

In Step 2, Compound (III) is treated in a solvent in the presence of a base, whereby, for example, ethyl 6,7-difluoro-1-[2-(S)-fluoro-1-(R)-cyclopropyl]-1,4-dihydro-8-methyl-4-oxoquinoline-3-carboxylate, which is included in the compounds represented by the formula (IV), can be produced.

The compound of the following formula (II) to be used in Step 1 will be explained.

R⁵-NH₂ (II)

Substituent R⁵ represents an alkyl group having 1 to 6 carbon atoms, an alkenyl group having 2 to 6 carbon atoms, a halogenoalkyl group having 1 to 6 carbon atoms, a cycloalkyl group having 3 to 6 carbon atoms, a halogenocycloalkyl group having 3 to 6 carbon atoms, an alkoxyl group having 1 to 6 carbon atoms, an alkylamino group having 1 to 6 carbon atoms, an aryl group which may have substituent(s), or a heteroaryl group which may have substituent(s).

In the definitions, ethyl group is particularly preferred as the alkyl group having 1 to 6 carbon atoms. As the alkenyl group having 2 to 6 carbon atoms, vinyl group or 1-isopropenyl group is preferred. As the halogenoalkyl group having 1 to 6 carbon atoms, 2-fluoroethyl group is preferred. As the cycloalkyl group having 3 to 6 carbon atoms, cyclopropyl group is preferred. Fluorine atom is preferred as the halogen atom of the halogenocycloalkyl group having 3 to 6 carbon atoms, and fluorocyclopropyl group is particularly preferred as the halogenocycloalkyl group.

As the alkoxyl group having 1 to 6 carbon atoms, methoxy group is preferred.

As the alkylamino group having 1 to 6 carbon atoms, methylamino group is preferred.

As the aryl group which may have substituent(s), there are mentioned phenyl groups and the like having 1 to 3 substituents selected from the substituent group consisting of, for example, halogen atoms such as fluorine atom, chlorine atom and bromine atom, hydroxyl group, amino group, nitro group, alkyl groups having 1 to 6 carbon atoms and alkoxyl groups having 1 to 6 carbon atoms, and preferred are phenyl group, 2-fluorophenyl group, 4-fluorophenyl group, 2,4-difluoro group, 2-fluoro-4-hydroxyphenyl group, 3-amino-4,6-difluorophenyl group, and 4,6-difluoro-3-methylaminophenyl group.

As the heteroaryl group which may have substituent(s), substituents derived from 5-membered or 6-membered ring aromatic heterocyclic compounds containing one or more heteroatoms selected from nitrogen atom, oxygen atom and sulfur atom can be exemplified. Specifically, pyridyl group, pyrimidyl group, and the like may be exemplified. As the substituents on these rings, alkyl groups, halogen atoms, and the like are preferred. As the heteroaryl group which may have substituent(s), particularly preferred is 5-amino-2,4-difluoropyridyl group.

As Substituent R⁵, preferred is a cycloalkyl group or a halogenocycloalkyl group. Among them, cyclopropyl group or a 2-halogenocyclopropyl group is preferred. Fluorine atom is preferred as the halogen atom. As the 2-fluorocyclopropyl group, particularly preferred is the one having (1R,2S) configuration.

For obtaining Compound (IV) composed of a single isomer, Compound (II) composed of a single isomer is to be reacted. Compound (II) composed of a single isomer, for example, cis-2-fluorocyclopropylamine composed of a single isomer can be produced according to the method disclosed in JP-A-2-231475.

Compound (II) is a commercially available compound or a compound obtained according to the method ordinarily used in this field, and is suitably used for the reaction in an amount of about 1 to 1.2 equivalents to Compound (I).

The solvent usable in Step 1 may be any solvent unless it inhibits the reaction, and examples thereof may include alcohol solvents such as methanol, ethanol, n-propanol and n-butanol; halogenated hydrocarbon solvents such as chloroform, methylene chloride and dichloroethane; ether solvents such as tetrahydrofuran, diethyl ether, 1,4-dioxane and dimethoxyethane; aromatic hydrocarbon solvents such as benzene, toluene and xylene; aprotic polar solvents such as acetonitrile, N,N-dimethylformamide and dimethylsulfoxide; and the like. These may be used as a mixture.

Reaction temperature may be usually from -60°C to 50°C, and the reaction may be preferably carried out in the range of -20°C to 30°C. Reaction time may be in the range of 30 minutes to 48 hours, and the reaction is usually completed within the range of about 30 minutes to 4 hours.

When Compound (II) forms a salt with an inorganic acid or an organic acid, about 1 to 1.5 equivalents of a base may be added to the reaction mixture for the purpose of converting Compound (II) into a free amine compound. The base may be any amine unless it inhibits the reaction, but a tertiary organic base is preferred and, for example, triethylamine may be exemplified.

The following will explain Step 2. The base usable in Step 2 may include potassium carbonate, sodium hydride, potassium t-butoxide, and the like.

The solvent usable in Step 2 may be any solvent as far as it is inert under the reaction conditions, and examples thereof may include ether solvents such as tetrahydrofuran, diethyl ether, 1,4-dioxane and dimethoxyethane; aromatic hydrocarbon solvents such as benzene, toluene and xylene; aprotic polar solvents such as acetonitrile, N,N-dimethylformamide and dimethylsulfoxide; or mixtures thereof.

Reaction temperature may be usually from icecooling to 150°C, and the reaction may be preferably carried out in the range of 20°C to 100°C. Reaction time may be in the range of 30 minutes to 48 hours, and the reaction is usually completed within the range of about 30 minutes to 20 hours. In this reaction, a catalyst may be employed, if necessary. For example, a phase transfer catalyst such as a crown ether, tetrabutylammonium bromide or benzyltriethylammonium bromide may be employed.

Step 1 and Step 2 may be carried out in the same reaction vessel as in a successive reaction. But, as the method for isolating Compound (IV) formed after completion of the reaction, an ordinary method may be mentioned wherein an acidic aqueous solution such as hydrochloric acid is added dropwise to the reaction mixture to adjust it to a weakly acidic solution, and then the solution is extracted with a water-insoluble solvent, followed by removal of the solvent through concentration, or crystals precipitated are collected by filtration. In the case of carrying out further purification, a general purification method such as column chromatography, recrystallization, or heated slurry may be applied, whereby the product can be isolated as a pure substance.

By the way, each reaction step is explained according to the scheme, but more specific processes are explained in Examples.

In the processes of the present invention, there are a process comprising a series of steps wherein chemical transformation is carried out starting with a compound having nitro group beforehand and a process comprising a series of steps wherein transformation is carried out starting with a compound having no nitro group and nitro group is introduced therebetween. The following will explain the latter process. This process is preferable especially for the production of a compound having a halogenocycloalkyl group at the 1- position.

First, the following will describe each substituent of the compound represented by the formula (VII) : 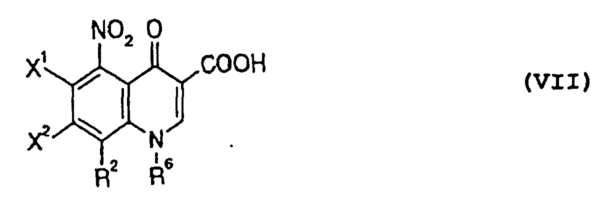 (wherein R² represents an alkyl group having 1 to 6 carbon atoms,
R⁶ represents a halogenocycloalkyl group having 3 to 6 carbon atoms,
X¹ represents a halogen atom or hydrogen atom, and
X² represents a leaving group.).

Substituent R² is an alkyl group having 1 to 6 carbon atoms, and examples of the alkyl group include methyl group, ethyl group, n-propyl group and isopropyl group. Among them, methyl group is preferred.

Substituent R⁶ represents a halogenocycloalkyl group having 3 to 6 carbon atoms. Fluorine atom is preferred as the halogen atom of the halogenocycloalkyl group, and as the halogenocycloalkyl group, preferred is a fluorocycloalkyl group and particularly preferred is a fluorocyclopropyl group. Among the fluorocylopropyl group, 2-fluorocylopropyl group is preferred. As the 2-fluorocyclopropyl group, particularly preferred is the one having (1R,2S) configuration.

Substituent X¹ represents a halogen atom or hydrogen atom, but in the case of a halogen atom, fluorine atom is preferred. Substituent X¹ is preferably fluorine atom or hydrogen atom.

Substituent X² means a leaving group. The leaving group includes halogen atoms, phenylsulfonyl groups which may have substituent(s), alkylsulfonyl groups having 1 to 3 carbon atoms which may have substituent(s), and the like. By the way, the leaving group is not limited thereto as far as it is the substituent known in this field as one having a function as a leaving group.

The halogen atoms are preferably fluorine atom, chlorine atom, and bromine atom. Examples of the phenylsulfonyl groups which may have substituent(s) include benzenesulfonyl group and p-toluenesulfonyl group, and examples of the alkylsulfonyl groups having 1 to 3 carbon atoms which may have substituent(s) include methanesulfonyl group, trifluoromethanesulfonyl group, and the like.

Substituent X² is preferably fluorine atom, chlorine atom and bromine atom, and particularly preferred is fluorine atom.

The following scheme shows a process for producing a 5-amino-1-halogenocycloalkyl-1,4-dihydro-8-alkyl-4-oxoquinoline-3-carboxylic acid derivative, through Compound (VII), for example, a 1-halogenocycloalkyl-1,4-dihydro-8-alkyl-5-nitro-4-oxoquinoline-3-carboxylic acid derivative as an intermediate for the production. 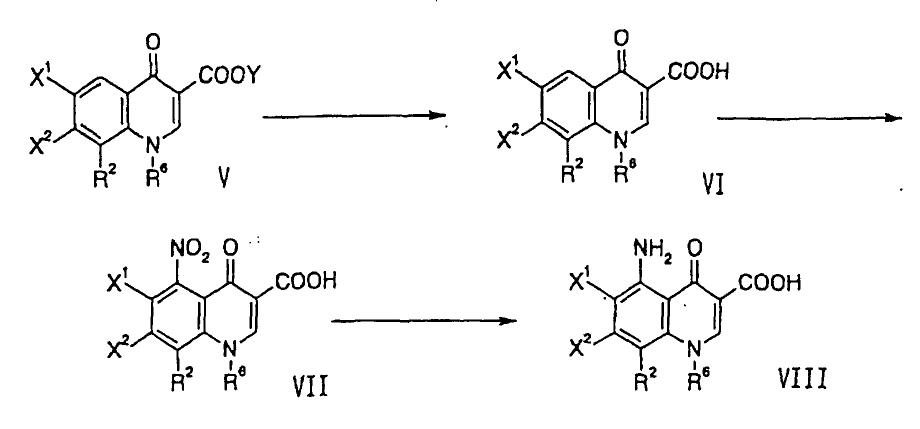 (in the scheme, R² represents an alkyl group having 1 to 6 carbon atoms,
R⁶ represents a halogenocycloalkyl group having 3 to 6 carbon atoms,
X¹ represents a halogen atom or hydrogen atom, and
X² represents a leaving group.)

Hereinafter, each reaction will be explained in detail by exemplifying the process for producing 5-amino-6,7-difluoro-1-[2-(S)-fluoro-1-(R)-cyclopropyl]-1,4-dihydro-8-methyl-4-oxoquinoline-3-carboxylic acid which is included in 5-amino-1-halogenocycloalkyl-1,4-dihydro-8-alkyl-4-oxoquinoline-3-carboxylic acid derivatives, but the process of the present invention is not limited to this example.

First, the step of cleavage of the ester is explained. In this step, any method for the cleavage of an ester ordinarily used in this field may be applied, and the following will explain hydrolysis as an example.

A 1,4-dihydro-8-methyl-4-oxoquinoline-3-carboxylic acid derivative can be produced by hydrolyzing 1,4-dihydro-8-methyl-4-oxoquinoline-3-carboxylic acid ester derivative. The detail is disclosed in the publication of Japanese Patent No. 2717597.

Next, the step of nitration is explained. This step is a step of treating a 1,4-dihydro-8-alkyl-4-oxoquinoline-3-carboxylic acid derivative represented by the formula (VI) with a nitrating agent, wherein a 1,4-dihydro-8-alkyl-5-nitro-4-oxoquinoline-3-carboxylic acid derivative represented by the formula (VII) can be produced.

The nitrating agent usable herein includes nitric acid, mixed acid (a mixture of nitric acid and sulfuric acid), metal nitrates such as potassium nitrate and sodium nitrate, acetyl nitrate, dinitrogen pentoxide, nitronium salts, and the like which are employed for ordinary nitration of aromatic hydrocarbon compounds.

As the conditions for the nitration, any of the conditions commonly known may be applied unless there is a possibility that the compound participating in the reaction is unnecessarily decomposed. Among the methods for nitration, a method of using a nitronium salt in the presence of an organic solvent is particularly preferred as a method applicable to the process of the invention.

Now, the following will explain the nitronium salt represented by the following formula, usable in the nitration step, and the nitration using the same. The nitronium salt compound is represented by the following formula:

(NO₂)⁺Z⁻

(wherein Z⁻ represents a counter anion of nitronium cation).

Examples of the counter anion Z of nitronium cation include (BF₄)⁻; (PF₄)⁻; (AsF₆)⁻; (SbF₆)⁻; (AuF₄)⁻; (CF₃SO₃)⁻. Among them, preferred are (BF₄)⁻; (CF₃SO₃)⁻.

Namely, as the nitronium salt, preferred is nitronium tetrafluoroborate or nitronium trifluoromethanesulfonate. Nitronium tetrafluoroborate is commercially available and nitronium trifluoromethanesulfonate can be easily prepared from trifluoromethanesulfonic acid and nitric anhydride or potassium nitrate at the time when needed. These nitronium salts may be used in an amount of about 1 to 10 equivalents to the compound of the formula (VI).

The reaction between Compound (VI) and the nitronium salt may be suitably carried out in the presence of an organic solvent. When water is present in the reaction system, there is a possibility that the nitronium salt decomposes, so that it is preferable to use a substantially water-free solvent in order to prevent the decomposition. In addition, for the purpose of preventing the contamination of water into the system, it is preferable to carry out the reaction under a dry nitrogen gas or inert gas atmosphere.

Examples of the solvent usable include aliphatic hydrocarbon solvents such as pentane, hexane and heptane; ether solvents such as tetrahydrofuran, diethyl ether and dimethoxyethane; halogenated hydrocarbon solvents such as methylene chloride and chloroform; aprotic polar solvents such as sulfolane, dimethylsulfoxide, nitromethane, acetonitrile, N,N-dimethylformamide and N,N-dimethylacetamide; water-free acids such as trifluoromethanesulfonic acid and conc. sulfuric acid; or mixtures thereof. Among them, in the case that nitronium tetrafluoroborate is used, it is preferable to use sulfolane, nitromethane and acetonitrile, and in the case of nitronium trifluoromethanesulfonate, it is preferable to use methylene chloride.

Reaction temperature may be usually from -78°C to 100°C, and the reaction may be preferably carried out in the range of -30°C to 60°C. Reaction time may be in the range of 15 minutes to 96 hours, and preferably 30 minutes to 48 hours.

As the method for isolating the product, i.e., Compound (VII) after completion of the nitration step, a method wherein after concentration of the reaction mixture or without concentration, a water-insoluble organic solvent and water are added and the whole is extracted, followed by removal of the solvent through concentration, or crystals precipitated are collected by filtration. In the case of carrying out further purification, the product can be isolated as a pure substance by applying column chromatography, recrystallization, or heated slurry.

The step of reduction is explained. This step is a step of reducing the nitro group at the 5-position of Compound (VII), wherein a 5-amino-1-halogenocycloalkyl-1,4-dihydro-8-alkyl-4-oxoquinoline-3-carboxylic acid derivative represented by the formula (VIII) can be produced.

The reduction may be carried out according to a method ordinarily known for the production of an aromatic primary amine by reducing an aromatic nitro compound. For example, a method of treatment with zinc or tin under acidic conditions or a method of catalytic reduction ordinarily known in this field may be mentioned. As the method of catalytic reduction, catalytic hydrogenation is often carried out using a transition metal complex such as platinum, Raney-nickel, platinum black (Pt-C), platinum oxide, palladium-carbon (Pd-C) or ruthenium, or the like as a catalyst.

The solvent to be used herein includes acetic acid, water, methanol, ethanol, N,N-dimethylformamide, or the like. The solvent usable in the reduction with a metal such as zinc includes hydrochloric acid, acetic acid, hydrobromic acid, or the like.

As the method for isolating the product, i.e., Compound (VIII) after completion of the reduction, an ordinary method may be applicable wherein after concentration of the reaction mixture or without concentration, a water-insoluble organic solvent and water are added and the product is extracted, followed by removal of the solvent through concentration, or crystals precipitated are collected by filtration. In the case of carrying out further purification, the product can be isolated as a pure substance by applying a general purifying method such as column chromatography, recrystallization, or heated slurry.

By the way, each reaction step is explained according to the scheme, but will be more specifically explained in Examples

### BEST MODE FOR CARRYING OUT THE INVENTION

The following will explain the present invention more specifically with reference to Examples and Comparative Examples, but the invention is not limited these examples.

### Example 1

### Ethyl 2-(2,4,5-trifluoro-3-methyl-6-nitrobenzoyl)-3-dimethylaminoacrylate

Into toluene (50 ml) was dissolved 2,4,5-trifluoro-3-methyl-6-nitrobenzoic acid (7.413 g; 31.53 mmol), and then N,N-dimethylformamide (0.1 ml) and thionyl chloride (2990 µl; 40.99 mmol) were added thereto, followed by 4 hours of stirring on an oil bath of 80°C. After cooling, the reaction solution was concentrated under reduced pressure. Toluene was added to the residue and concentration under reduced pressure was repeated. Then, the resulting residue was dissolved into tetrahydrofuran (10 ml). The solution was added dropwise at room temperature to a solution of ethyl 3-dimethylaminoacrylate (4.141 g; 33.11 mmol) and triethylamine (4615 µl; 33.11 mmol) dissolved in tetrahydrofuran (15 ml). After completion of the addition, the reaction solution was heated under reflux for 10 hours. After completion of the reaction, the solvent was removed by evaporation under reduced pressure and the resulting residue was dissolved into methylene chloride (150 ml), and the solution was washed with water (150 ml x 2) and saturated brine (150 ml), successively. Then, the organic layer was dried over anhydrous sodium sulfate. After filtration, the filtrate was concentrated under reduced pressure and the resulting residue was subjected to short silica gel column chromatography (at first, eluted with toluene), to obtain red-yellow amorphous title compound (6.180 g, 54.4%) from fractions eluted with n-hexane/ethyl acetate = 1/1. ¹H-NMR (400 MHz, CDCl₃) δ: 1.06 (3H, t, J=7.08Hz), 2.28 (3H, t, J=1.95Hz), 3.08 (3H, s), 3.40 (3H, s), 4.03 (2H, q, J=7.08Hz), 8.01 (1H, s).
MS: m/z: 360 (M⁺).

### Example 2

### Process for producing ethyl 1-cyclopropyl-6,7-difluoro-1,4-dihydro-8-methyl-5-nitro-4-oxoquinoline-3-carboxylate

Ethyl 2-(2,4,5-trifluoro-3-methyl-6-nitrobenzoyl) -3-dimethylaminoacrylate (1.000 g; 2.776 mmol) was dissolved into tetrahydrofuran (5 ml), and cyclopropylamine (209 µl; 3.05 mmol) was added dropwise under stirring at room temperature. After 4 hours of stirring of the reaction solution at room temperature, N,N-dimethylformamide (5 ml) and potassium carbonate (1.151 g; 8.323 mmol) were added successively thereto, and the reaction suspension was stirred at room temperature for 16 hours, followed by 1 hour of stirring on an oil bath of 60°C. After cooling of the reaction solution with ice, 2 mol/l hydrochloric acid was gradually added dropwise under stirring to adjust pH to about 3. After 1 hour of stirring at room temperature, the precipitated crystals were collected by filtration, and the crystals were washed with ethanol and diethyl ether and then dried under reduced pressure at room temperature for 1 hour. The resulting crude crystals were suspended in ethyl acetate (5 ml), and after 30 minutes of stirring on an oil bath of 80°C, the whole was cooled to room temperature. The crystals were collected by filtration and then, washed with cold ethyl acetate, cold ethanol, and diethyl ether. Drying at 70°C under reduced pressure afforded pale yellow powdery title compound (749 mg, 77.6%). The following shows ¹H-NMR data of this product, the data being coincident with the data of title compound synthesized according to the process disclosed in JP-A-7-309864.
¹H-NMR (400 MHz, CDCl₃) δ: 1.00-1.04 (2H, m), 1.26-1.30 (2H, m), 1.37 (3H, t, J=7.08Hz), 2.80 (3H, d, J=3.17Hz), 3.96-4.01 (1H, m), 4.36 (2H, q, J=7.08Hz), 8.67 (1H, s). MS: m/z: 352 (M⁺).

### Example 3

### Process for producing ethyl 6,7-difluoro-1-[2-(S)-fluoro-1-(R)-cyclopropyl]-1,4-dihydro-8-methyl-5-nitro-4-oxoquinoline-3-carboxylate

Ethyl 2-(2,4,5-trifluoro-3-methyl-6-nitrobenzoyl)-3-dimethylaminoacrylate (1.000 g; 2.776 mmol) was dissolved into tetrahydrofuran (4 ml), and (1R,2S)-2-fluorocyclopropylamine p-toluenesulfonate (755.0 mg; 3.053 mmol) was added. Then, under stirring at -15°C, a solution of triethylamine (511 µl; 3.66 mmol) dissolved in tetrahydrofuran (2 ml) was added dropwise. After 4 hours of stirring of the reaction solution at room temperature, N,N-dimethylformamide (5 ml) and potassium carbonate (1.151 g; 8.323 mmol) were added successively thereto, and the reaction suspension was stirred at room temperature for 17 hours, followed by 1 hour of stirring on an oil bath of 60°C. After cooling of the reaction solution with ice, 2 mol/l hydrochloric acid was gradually added dropwise under stirring to adjust pH to about 3. After 1 hour of stirring at room temperature, the precipitated crystals were collected by filtration, and the crystals were washed with ethanol and diethyl ether and then dried under reduced pressure at room temperature for 1 hour. The resulting crude crystals were suspended into ethyl acetate (5 ml), and after 30 minutes of stirring on an oil bath of 80°C, the whole was cooled to room temperature. The crystals were collected by filtration and then, washed with cold ethyl acetate, cold ethanol, and diethyl ether. Drying at 70°C under reduced pressure afforded pale yellow powdery title compound (742 mg, 72.2%). The following shows ¹H-NMR data of this product, the data being coincident with the data of title compound synthesized according to the process disclosed in JP-A-8-277284. ¹H-NMR (400 MHz, CDCl₃) δ: 1.37 (3H, t, J=7.08Hz), 1.36-1.46 (1H, m), 1.59-1.68 (1H, m), 2.75 (3H, d, J=3.17Hz), 3.86-3.91 (1H, m), 4.37 (2H, q, J=7.08Hz), 4.80-4.82 and 4.96-4.98 (1H, dm, J=69.09Hz), 8.56 (1H, d, J=2.93Hz).

### Example 4

### Ethyl 2-(2,4,5-trifluoro-3-methylbenzoyl)-3-dimethylaminoacrylate

Into toluene (100 ml) was dissolved 2,4,5-trifluoro-3-methylbenzoic acid (13.22 g; 69.83 mmol), and then N,N-dimethylformamide (0.2 ml) and thionyl chloride (7593 µl; 104.1 mmol) were added thereto, followed by 12 hours of stirring on an oil bath of 80°C. After cooling, the reaction solution was concentrated under reduced pressure. Toluene was added to the residue and the concentration under reduced pressure was repeated. Then, the resulting residue was dissolved into tetrahydrofuran (20 ml). The solution was added dropwise under icecooling to a solution of ethyl 3-dimethylaminoacrylate (11.99 g; 83.80 mmol) and triethylamine (12.26 ml; 87.93 mmol) dissolved in tetrahydrofuran (30 ml). After completion of the addition, the reaction solution was heated under reflux for 10 hours. After completion of the reaction, the reaction mixture was filtrated to remove triethylamine hydrochloride (washing with diethyl ether) and the filtrate was concentrated under reduced pressure. The resulting residue was subjected to short silica gel column chromatography to obtain pink powdery title compound (19.07 g, 86.6%) from fractions eluted with n-hexane/ethyl acetate = 1/1. ¹H-NMR (400 MHz, CDCl₃) δ: 0.99 (3H, t, J=7.10Hz), 2.21 (3H, t, J=2.20Hz), 2.86 (3H, s), 3.30 (3H, s), 4.01 (2H, q, J=7.10Hz), 7.28 (1H, brs), 7.76 (1H, s).

### Example 5

### Process for producing ethyl 6,7-difluoro-1-[2-(S)-fluoro-1- (R)-cyclopropyl]-1,4-dihydro-8-methyl-4-oxoquinoline-3-carboxylate

Ethyl 2-(2,4,5-trifluoro-3-methylbenzoyl)-3-dimethylaminoacrylate (3.153 g; 10.00 mmol) was dissolved into tetrahydrofuran (30 ml), and then (1R,2S)-2-fluorocyclopropylamine p-toluenesulfonate (2.720 g; 11.00 mmol) was added. Then, under stirring at -15°C, a solution of triethylamine (1549 µl; 11.11 mmol) dissolved in tetrahydrofuran (10 ml) was added dropwise. After 4 hours of stirring of the reaction solution at room temperature, potassium carbonate (2.073 g; 15.00 mmol) and tetrabutylammonium chloride (139 mg; 0.500 mmol) were added thereto, and the reaction suspension was stirred at room temperature for 16 hours and heated under reflux. After cooling of the reaction solution, tetrahydrofuran was removed by evaporation under reduced pressure. Under stirring of the residue with ice-cooling, 2 mol/l hydrochloric acid was gradually added dropwise thereto under stirring to adjust pH to about 3. After 15 minutes of stirring at room temperature, the whole was extracted with dichloromethane (60 ml x 3). After drying over anhydrous sodium sulfate, the extract was filtrated and the filtrate was concentrated under reduced pressure. The resulting crude crystals were purified by recrystallization from ethanol to obtain title compound (2.410 g, 74.1%) as colorless crystals. The following shows ¹H-NMR data of this product, the data being coincident with the data disclosed in Japanese Patent No. 2714597.
¹H-NMR (400 MHz, CDCl₃) δ: 1.37 (3H, t, J=7.08Hz), 1.36-1.46 (1H, m), 1.59-1.68 (1H, m), 2.75 (3H, d, J=3.17Hz), 3.86-3.91 (1H, m), 4.37 (2H, q, J=7.08Hz), 4.80-4.82 and 4.96-4.98 (1H, dm, J=69.09Hz), 8.56 (1H, d, J=2.93Hz).

### Reference Example 1

### 6,7-Difluoro-1-[2-(S)-fluoro-1-(R)-cyclopropyl]-1,4 dihydro-8-methyl-4-oxoquinoline-3-carboxylic acid

Into acetic acid (40 ml) was dissolved ethyl 6,7-difluoro-1-[2-(S)-fluoro-1-(R)-cyclopropyl]-1,4-dihydro-8-methyl-4-oxoquinoline-3-carboxylate (3.003 g; 9.232 mmol), and then conc. hydrochloric acid (40 ml) was added thereto, followed by 4 hours of heating under reflux. After cooling, the reaction solution was poured into excess ice-water and the precipitated crystals were collected by filtration. The collected crystals were washed with excess water, and then washed with cold ethanol and diethyl ether, successively. Drying under reduced pressure afforded white powdery title compound (2.541 g, 92.8%). The following shows ¹H-NMR data of this product, the data being coincident with the data disclosed in Japanese Patent No. 2714597.
¹H-NMR (400 MHz, CDCl₃) δ: 1.36-1.87 (1H, m) , 2.80 (3H, d, J=3.20Hz), 3.86-4.00 (1H, m), 4.80-4.84 and 4.96-5.00 (1H, dm, J=65.07Hz), 8.15 (1H, dd, J=15.5Hz), 8.60 (1H, d, J=2.93Hz).

### Example 6

### 6,7-Difluoro-1-[2-(S)-fluoro-1-(R)-cyclopropyl]-1,4-dihydro-8-methyl-5-nitro-4-oxoquinoline-3-carboxylic acid

Under a nitrogen atmosphere, nitronium tetrafluoroborate (535 mg; 4.04 mmol) was dissolved into nitromethane (30 ml). Under ice-cooling, 6,7-difluoro-1-[2-(S)-fluoro-1-(R)-cyclopropyl]-1,4-dihydro-8-methyl-4-oxoquinoline-3-carboxylic acid (1.00 g; 3.37 mmol) was added thereto and the whole was stirred at room temperature for 16 hours under a nitrogen atmosphere. The reaction solution was poured into ice-water (50 ml) and the whole was extracted with chloroform (60 ml x 2). The organic layer was washed with saturated brine (50 ml) and dried over anhydrous sodium sulfate. After filtration, the filtrate was concentrated under reduced pressure. The residue was subjected to short silica gel column chromatography (eluted with lower layer of chloroform/methanol/water = 20/3/1), to obtain title compound (490 mg, 42.2%) as a pale yellow powder. Also, 6,7-difluoro-1-[2-(S)-fluoro-1-(R)-cyclopropyl]-1,4-dihydro-8-methyl-4-oxoquinoline-3-carboxylic acid (400 mg, 40.0%) was recovered. ¹H-NMR (400 MHz, CDCl₃) δ: 1.36-1.46 (1H, m), 1.59-1.68 (1H, m), 2.84 (3H, d, J=3.41Hz), 4.02-4.09 (1H, m), 4.98-5.02 and 5.11-5.15 (1H, dm, J=65.13Hz), 8.85 (1H, d, J=2.69Hz), 14.28 (s, 1H).

### Example 7

### 6,7-Difluoro-1-[2-(S)-fluoro-1- (R)-cyclopropyl]-1,4-dihydro-8-methyl-5-nitro-4-oxoquinoline-3-carboxylic acid

Under a nitrogen atmosphere, 6,7-difluoro-1-[2-(S)-fluoro-1-(R)-cyclopropyl]-1,4-dihydro-8-methyl-4-oxoquinoline-3-carboxylic acid (500 mg; 1.67 mmol) was dissolved into sulfolane (8 ml). Under ice-cooling, 0.5M sulfolane solution of nitronium tetrafluoroborate (7.40 ml; 3.34 mmol) was added dropwise thereto. The reaction solution was stirred at room temperature for 24 hours. Then, the reaction solution was poured into ice-water (50 ml) and extracted with chloroform (50 ml x 2). The organic layer was washed with saturated brine (50 ml x 3) and dried over anhydrous sodium sulfate. After filtration, the filtrate was concentrated under reduced pressure. The residue was subjected to short silica gel column chromatography (eluted with lower layer of chloroform/methanol/water = 20/3/1), to obtain title compound (267 mg, 46.0%) as a pale yellow powder. Also, 6,7-difluoro-1-[2-(S)-fluoro-1-(R) -cyclopropyl)-1,4-dihydro-8-methyl-4-oxoquinoline-3-carboxylic acid (206 mg, 41.1%) was recovered.

### Example 8

### Process for producing 5-amino-6,7-difluoro-1-[2-(S)- fluoro-1-(R) -cyclopropyl] -1,4-dihydro-8-methyl-4-oxoquinoline-3-carboxylic acid

Into N,N-dimethylformamide (25 ml) was dissolved 6,7-difluoro-1-[2-(S)-fluoro-1-(R)-cyclopropyl]-1,4-dihydro-8-methyl-5-nitro-4-oxoquinoline-3-carboxylic acid (475 mg; 1.39 mmol). After addition of 10% palladium-carbon catalyst (moisture 50%) (200 mg), the whole was stirred at room temperature for 3 hours under a hydrogen atmosphere of normal pressure. The reaction mixture was filtrated through celite (washing with N,N-dimethylformamide), and then the filtrate was concentrated under reduced pressure. Ethanol (20 m) was added to the residue and the whole was stirred at room temperature. The precipitated crystals were collected by filtration, and washed with ethanol. Drying under reduced pressure afforded yellow powdery title compound (375 mg, 86.1%). The following shows ¹H-NMR data of this product, the data being coincident with the data disclosed in JP-A-8-277284.
¹H-NMR (400 MHz, CDCl₃) δ: 1.31-1.42 (1H, m), 1.53-1.68 (1H, m), 2.52 (3H, d, J=3.36Hz), 4.03-4.10 (1H, m), 4.85-5.93 and 5.05-5.10 (1H, dm, J=68.11Hz), 8.32 (1H, s).

### INDUSTRIAL APPLICABILITY

According to the process using a 3-dialkylamino-2-(3-methylbenzoyl)acrylic acid derivative and a 1,4-dihydro-8-methyl-5-nitro-4-oxoquinoline-3-carboxylic acid derivative provided by the present invention as intermediates for the production, a 5-amino-1,4-dihydro-8-methyl-4-oxoquinoline-3-carboxylic acid derivative which is an intermediate in the production of a fluoroquinolonecarboxylic acid-type synthetic antibacterial agent exhibiting excellent antibacterial activity, pharmacokinetics and safety can be produced in short steps and conveniently as compared with the conventional methods.

## Claims

1. A compound represented by the formula (I): 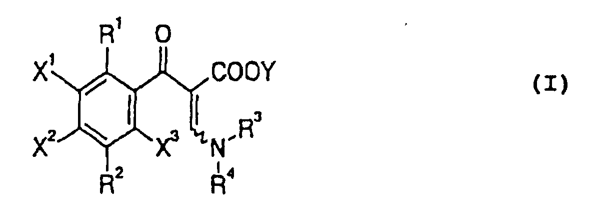 (wherein R¹ represents hydrogen atom or nitro group; R² represents an alkyl group having 1 to 6 carbon atoms; R³ and R⁴ represent each independently an alkyl group having 1 to 6 carbon atoms or may form a 5-membered or 6-membered cyclic structure in combination with the nitrogen atom to which they are bonded, and the cyclic structure may contain oxygen atom, nitrogen atom, sulfinyl group, or sulfonyl group as a ring-constituting element; X¹ represents a halogen atom or hydrogen atom; X² and X³ represent each independently a leaving group; and Y represents an alkyl group having 1 to 6 carbon atoms or an alkenyl group having 2 to 6 carbon atoms) and salt thereof.

2. The compound and salt thereof according to claim 1, wherein the leaving group is a halogen atom, a phenylsulfonyl group which may have substituent(s), or an alkylsulfonyl group having 1 to 3 carbon atoms which may have substituent(s).

3. The compound and salt thereof according to claim 1 or 2, wherein R¹ is nitro group.

4. The compound and salt thereof according to claim 1 or 2, wherein R¹ is hydrogen atom.

5. The compound and salt thereof according to any one of claims 1 to 4, wherein R² is methyl group.

6. The compound and salt thereof according to any one of claims 1 to 5, wherein X¹, X² and X³ are each fluorine atom.

7. A process for producing a compound represented by the formula (IV) : 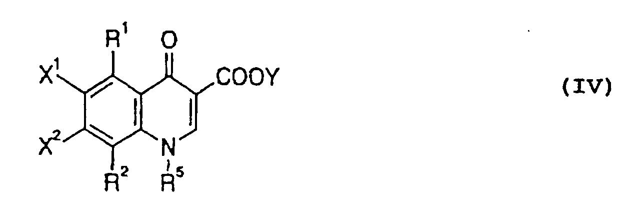 (wherein R¹, R², R⁵, X¹, X² and Y are each the same as defined above), which comprises reacting a compound represented by the formula (I) : 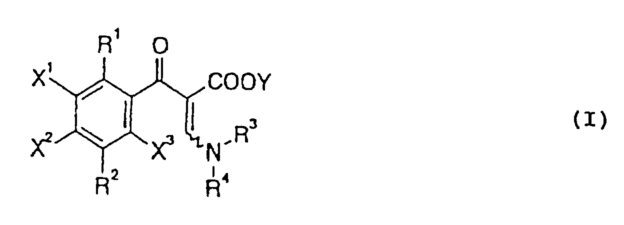 (wherein R¹ represents hydrogen atom or nitro group; R² represents an alkyl group having 1 to 6 carbon atoms; R³ and R⁴ represent each independently an alkyl group having 1 to 6 carbon atoms or may form a 5-membered or 6-membered cyclic structure in combination with the nitrogen atom to which they are bonded, and the cyclic structure may contain oxygen atom, nitrogen atom, sulfinyl group, or sulfonyl group as a ring-constituting element; X¹ represents a halogen atom or hydrogen atom; X² and X³ represent each independently a leaving group; and Y represents an alkyl group having 1 to 6 carbon atoms or an alkenyl group having 2 to 6 carbon atoms) or salt thereof, with a compound represented by the formula (II):
R⁵-NH₂ (II)
(wherein R⁵ represents an alkyl group having 1 to 6 carbon atoms, an alkenyl group having 2 to 6 carbon atoms, a halogenoalkyl group having 1 to 6 carbon atoms, a cycloalkyl group having 3 to 6 carbon atoms, a halogenocycloalkyl group having 3 to 6 carbon atoms, an alkoxyl group having 1 to 6 carbon atoms, an alkylamino group having 1 to 6 carbon atoms, an aryl group which may have substituent(s), or a heteroaryl group which may have substituent(s)) to obtain a compound represented by the formula (III): 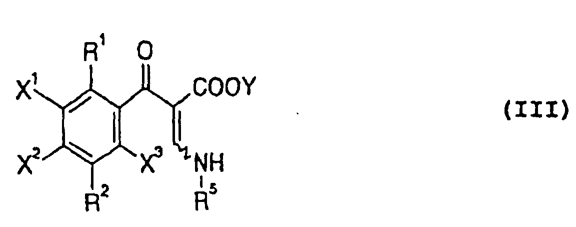 (wherein R¹, R², R⁵, X¹, X², X³ and Y are each the same as defined above) or a salt thereof, and treating the compound of the formula (III) with a base.

8. The process according to claim 7, wherein the leaving group is a halogen atom, a phenylsulfonyl group which may have substituent(s), or an alkylsulfonyl group having 1 to 3 carbon atoms which may have substituent(s).

9. The process according to claim 7 or 8, wherein R¹ is nitro group.

10. The process according to claim 7 or 8, wherein R¹ is hydrogen atom.

11. The process according to any one of claims. 7 to 10, wherein R² is methyl group.

12. The process according to any one of claims 7 to 11, wherein X¹, X² and X³ are each fluorine atom.

13. The process according to any one of claims 7 to 11, wherein X¹, X² and X³ are each fluorine atom and R⁵ is cyclopropyl group or a halogenocyclopropyl group.

14. The process according to any one of claims 7 to 11, wherein X¹, X² and X³ are each fluorine atom and R⁵ is 2-(S)-fluoro-1-(R)-cyclopropyl group.

15. A compound represented by the formula (VII): 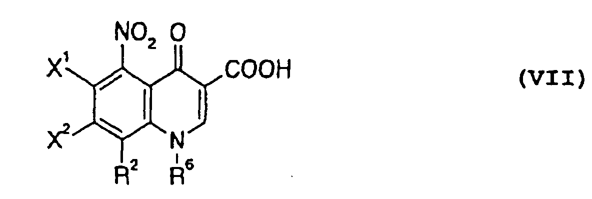 (wherein R² represents an alkyl group having 1 to 6 carbon atoms, R⁶ represents a halogenocycloalkyl group having 3 to 6 carbon atoms, X¹ represents a halogen atom or hydrogen atom, and X² represents a leaving group) and a salt thereof.

16. The compound and salt thereof according to claim 15, wherein the leaving group is a halogen atom, a phenylsulfonyl group which may have substituent(s), or an alkylsulfonyl group having 1 to 3 carbon atoms which may have substituent(s).

17. The compound and salt thereof according to claim 15 or 16, wherein R² is methyl group.

18. The compound and salt thereof according to any one of claims 15 to 17, wherein X¹ and X² are each fluorine atom.

19. The compound and salt thereof according to any one of claims 15 to 17, wherein X¹ and X² are each fluorine atom and R⁶ is 2-(S)-fluoro-1-(R)-cyclopropyl group.

20. A process for producing a compound represented by the formula (VII): 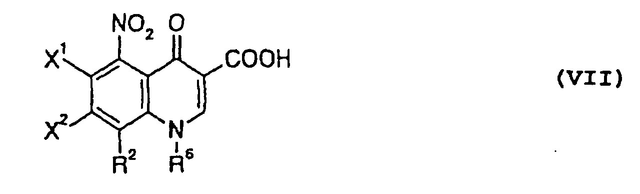 (wherein R², R⁶, X¹ and X² are each the same as defined above) or salt thereof,
which comprises converting the carboxylic acid ester at the 3-position in a compound represented by the formula (V): 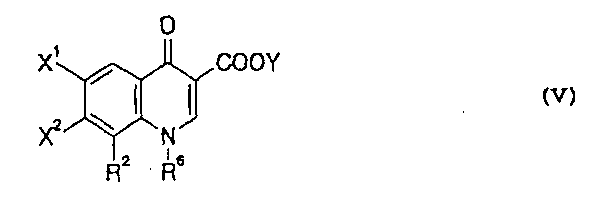 (wherein R² represents an alkyl group having 1 to 6 carbor atoms, R⁶ represents a halogenocycloalkyl group having 3 to 6 carbon atoms, X¹ represents a halogen atom or hydrogen atom, X² represents a leaving group, and Y represents an alkyl group having 1 to 6 carbon atoms or an alkenyl group having 2 to 6 carbon atoms) or salt thereof, into carboxyl group to obtain a compound represented by the formula (VI): 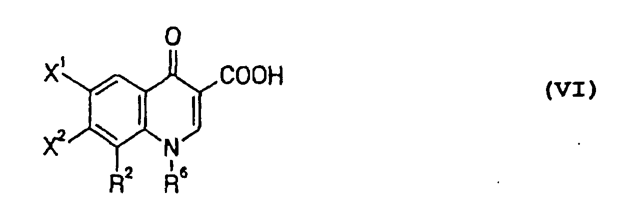 (wherein R², R⁶, X¹ and X² are each the same as defined above),
and treating the compound of the formula (VI) with a nitrating agent.

21. A process for producing a compound represented by the formula (VII): 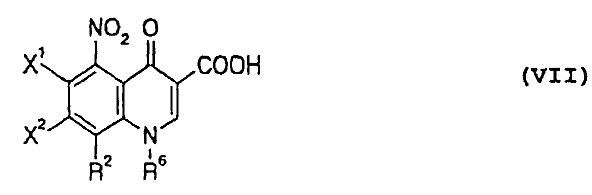 (wherein R², R⁶, X¹ and X² are each the same as defined above) or salt thereof,
which comprises treating a compound represented by the formula (VI): 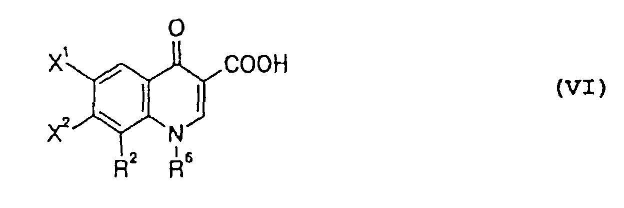 (wherein R² represents an alkyl group having 1 to 6 carbon atoms, R⁶ represents a halogenocycloalkyl group having 3 to 6 carbon atoms, X¹ represents a halogen atom or hydrogen atom, and X² represents a leaving group) or a salt thereof, with a nitrating agent.

22. The process according to claim 20 or 21, wherein the nitrating agent is nitric acid, a mixture of nitric acid and sulfuric acid, a metal nitrate, acetyl nitrate, dinitrogen pentoxide, or a nitronium salt.

23. The process according to claim 20 or 21, wherein the nitrating agent is a nitronium salt.

24. A process for producing a compound represented by the formula (VIII) 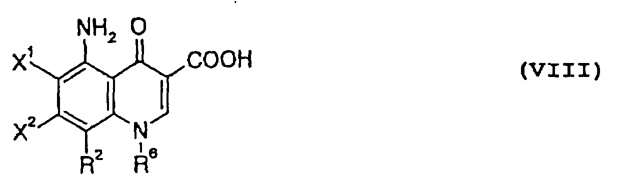 (wherein R², R⁶, X¹ and X² are each the same as defined above) or salt thereof,
which comprises converting the nitro group of a compound represented by the formula (VII): 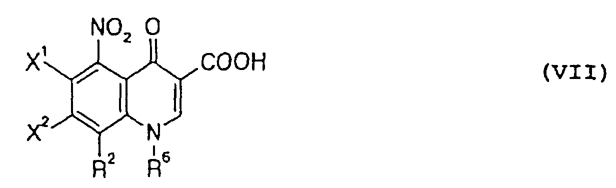 (wherein R² represents an alkyl group having 1 to 6 carbon atoms, R⁶ represents a halogenocycloalkyl group having 3 to 6 carbon atoms, X¹ represents a halogen atom or hydrogen atom, and X² represents a leaving group) or a salt thereof, into amino group.

25. The process according to any one of claims 20 to 24, wherein the leaving group is a halogen atom, a phenylsulfonyl group which may have substituent(s), or an alkylsulfonyl group having 1 to 3 carbon atoms which may have substituent(s).

26. The process according to any one of claims 20 to 25, wherein R² is methyl group.

27. The process according to any one of claims 20 to 26, wherein X¹ and X² are each fluorine atom.

28. The process according to any one of claims 20 to 26, wherein X¹ and X² are each fluorine atom and R⁶ is 2-(S)-fluoro-1-(R)-cyclopropyl group.

29. The compound and salt thereof according to any one of claims 1 to 6, wherein R³ and R⁴ are each an alkyl group having 1 to 6 carbon atoms.

30. The process according to any one of claims 7 to 14, wherein R³ and R⁴ are each an alkyl group having 1 to 6 carbon atoms.
